# EUROPEAN PATENT SPECIFICATION

(11) **EP 2 086 532 B1**
(45) Date of publication and mention of the grant of the patent: **13.07.2011**
(21) Application number: 07793743.1
(22) Date of filing: 29.08.2007
(51) Int. Cl.: A61K 31/35, A61P 3/10, A61P 3/04

(54) **USE FOR TREATING OBESITY AND DIABETES**
VERWENDUNG ZUR BEHANDLUNG VON FETTSUCHT UND DIABETES
UTILISATION D'UNE COMPOSITION POUR TRAITER L'OBÉSITÉ ET LE DIABÈTE

(30) Priority: 31.10.2006 KR 20060106362
(43) Date of publication of application: 12.08.2009
(73) Proprietor: Amorepacific Corporation, Seoul 140-777 (KR)
(72) Inventor: PARK, Hyun Woo, Annyang-si Gyeonggi-do 431-050 (KR); CHUN, Ji Young, Annyang-si Gyeonggi-do 431-762 (KR); SHIN, Eui Seok, Gwangju-si Gyeonggi-do 464-871 (KR); KIM, Yoo, Seongnam-si Gyeonggi-do 463-725 (KR); LEE, Tae Ryong, Yongin-si Gyeonggi-do 446-911 (KR); LEE, Sang Jun, Seongnam-si Gyeonggi-do 463-909 (KR)
(74) Representative: Ilgart, Jean-Christophe
(86) International application number: PCT/KR2007/004160
(87) International publication number: WO 2008/054059

(56) References cited:
- WO-A-00/45651
- WO-A1-2004/084885
- WO-A1-2004/093865
- YANG JI-YEON ET AL: "Effect of genistein with carnitine administration on lipid parameters and obesity in C57BI/6J mice fed a high-fat diet" JOURNAL OF MEDICINAL FOOD, vol. 9, no. 4, January 2006 (2006-01), pages 459-467, XP009122950 ISSN: 1096-620X
- DATABASE WPI Week 200644 Thomson Scientific, London, GB; AN 2006-428395 XP002546876 & KR 2005 035 218 A (AMOREPACIFIC CORP) 15 April 2005 (2005-04-15)
- ABDEL KADER M M ET AL: "EFFECT OF DL CARNITINE ACETYL-D L-BETA METHYL CHOLINE CHLORIDE AND GLYCINE BETAINE ON SOME PROCESSES OF CARBOHYDRATE METABOLISM OF HUMANS AND GOATS" ACTA BIOLOGICA ET MEDICA GERMANICA, AKAD.-VERLAG, DE, vol. 38, no. 11-12, 1 January 1979 (1979-01-01), pages 1485-1496, XP009122865 ISSN: 0001-5318
- GOODMAN-GRUEN DEBORAH ET AL: "Usual dietary isoflavone intake is associated with cardiovascular disease risk factors in postmenopausal women" JOURNAL OF NUTRITION, vol. 131, no. 4, April 2001 (2001-04), pages 1202-1206, XP002546875 ISSN: 0022-3166
- FU WENJIANG J ET AL: "Dietary L-arginine supplementation reduces fat mass in Zucker diabetic fatty rats" JOURNAL OF NUTRITION, WISTAR INSTITUTE OF ANATOMY AND BIOLOGY, PHILADELPHIA, PA, US, vol. 135, no. 4, 1 April 2005 (2005-04-01), pages 714-721, XP009123019 ISSN: 0022-3166
- KOBAYASHI-HATTORI KAZUO ET AL: "Effect of caffeine on the body fat and lipid metabolism of rats fed on a high-fat diet" BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 69, no. 11, 23 November 2005 (2005-11-23), pages 2219-2223, XP009122996 ISSN: 0916-8451

## Description

### [Technical Field]

The present invention relates to the use of a composition, containing isoflavone-containing soybean extract, L-carnitine, caffeine and arginine as active ingredients, for the treatment of obesity and diabetes.

### [Background Art]

In the human body, there are about 2 x 10¹⁰ fatty acids, which serve to store or release energy in the living mammalian body. Energy in these cells is stored and released according to complex regulatory mechanisms, and when the supply of energy is much larger than the demand, fatty acids are stored as neutral fats, and when energy is consumed, the neutral fats are decomposed into free fatty acids and glucoses. It is considered that obesity occurs when excessive energy is stored due to the unbalance of this process, and it is attributable to an increase in the size of fatty acids or in the number thereof.

Obesity occurring in 30-40% of modern persons is known to be a strong risk factor, which can cause hypertension, coronary artery diseases, type 2 diabetes and various forms of cancers. Particularly, obesity and diabetes are very closely connected with each other in the prevalent mechanisms.

Generally, obesity shows a decrease in insulin sensitivity with an increase in an increase in body fat, and particularly, the accumulation of abdominal fat is associated with glucose intolerance. Obesity is one of various causes of insulin resistance, and in some cases, type 2 diabetes do not occur in morbid obesity. However, in patients having type 2 diabetes, obesity and insulin resistance are closely correlated with each other, and thus, as obesity becomes severe, insulin resistance also becomes severe.

Dyslipidemia in type 2 diabetes is generally improved when blood glucose is regulated, but in some patients, it is not improved. The latter case is called "insulin resistance syndrome" or "central obesity syndrome". The most important feature of the insulin resistance syndrome is central obesity or visceral obesity. The central obesity and the visceral obesity cause insulin resistance and accompany hyperinsulinemia, hypertension or impaired glucose tolerance.

The induction of diabetes by obesity is currently considered as an important issue, and insulin sensitivity-improving agents, which can reduce insulin resistance in order to improve obesity and diabetes simultaneously, have been reported. Examples of the insulin sensitivity-improving agents include Xenical (Orlistat) and Reductil (Sibutramine), as obesity treatment drugs, the therapeutic effects of which were proven through long-term clinical trials of thiazolidinedione drugs and biguanide drugs on obesity patients having metabolic syndromes. However, such drugs shows anti-obesity effects through the mechanism of appetite inhibiting appetite and fat absorption rather than promoting the burning and decomposition of fat, and thus are not sufficient for solving insulin resistance. For this reason, such drugs cannot completely solve diabetes together with obesity, and in addition, the serious side effects thereof have been reported, so that the safety thereof is not yet established. Accordingly, there is a need to develop a novel substance, which shows an effect equal to or higher than that of the prior substances and, at the same time, is safer.

Thus, in view of various diseases acting as causes of diabetes resulting from obesity, it is evident that a decrease in body weight is more important than a simple decrease in bodyweight. Accordingly, it seems to be preferable to find out a method capable of inhibiting the accumulation of ingested fat and activating the burning of the fat, and in this point of view, a method capable of maintaining the expression level of adiponectin secreted from adipocytes while increasing the beta-oxidation of fatty acids can be an excellent target for anti-obesity and anti-diabetic effects.

The applicant filed obesity-related patent applications relating to promoting the reduction of body fat. The patent applications disclose that genistein and carnitine increase the expression of a carnitine palmitoyltransferase-1 (CPT-1), which is a key enzyme in the fatty acid degradation pathway, so as to promote the burning of body fat (Korean Patent Application No. 2003-0018559), and that a composition, containing theanine, caffeine, genistein and carnitine alone or in a mixture, shows excellent effects on lipolysis and cellulite removal (Korean Patent Application No. 2003-0098859). In addition, the patent application KR 2005/0019551 of the applicant discloses a food composition presenting excellent effect on suppressing or preventing obesity, said composition comprising 0,001 to 30% of genistein and 0,001 to 50% of carnitine. However, it is not known whether such compositions can promote the burning of fat and increase the expression level of adiponectin so as to improve diabetes caused by obesity.

Yang et al. 2006 (journal of Medicinal Food, vol. 9 pages 459-467) discloses that genistein with carnitine exerts anti-obesity effects, probably by modulating peroseisome proliferator-activated receptor-associated genes.

### [Disclosure]

### [Technical Problem]

Accordingly, the present inventors have conducted many studies and experiments in order to solve the above-described problems occurring in the prior art and, as a result, have developed an ideal anti-obesity and anti-diabetic composition, which contains components acting to promote lipolysis and fat burning, without containing components having diuretic action or appetite-suppressing action, so that the composition can improve obesity by removing excessive body fat, particularly, abdominal fat, through lipolysis and fat burning, and, at the same time, can treat diabetes by lowering blood glucose levels through an increase in the expression of adiponectin gene, which regulates insulin sensitivity.

Therefore, it is an object of the present invention to provide a composition for treating obesity and diabetes, which is effective in improving obesity by promoting the decomposition and burning of fat, accumulated in adipocytes, to reduce body fat, and at the same time, is effective in treating and improving diabetes by overcoming insulin resistance.

### [Technical Solution]

To achieve the above object, the present invention provides the use of a composition, containing isoflavone and L-carnitine as active ingredients, for the treatment of diabetes and obesity, the composition further containing caffeine and arginine as active ingredients.

Hereinafter, the present invention will be described in further detail.

The composition according to the present invention shows the effects of preventing and treating obesity and diabetes not only by reducing body weight and body fat, but also by improving diabetic syndromes, which are typical metabolic syndromes which can be induced due to obesity.

Isoflavone, which is contained in soybean in large amounts and is a vegetable hormone similar to a female hormone, was reported to show various physiological activities. Recently, it was reported to have various effects, for example, the effects of regulating fat metabolisms in adipocytes and reducing blood cholesterol levels.

Carnitine, which is synthesized in the liver or kidneys of normal persons and contained in red fishes in large amounts, is known to be an important component in oxidizing fat to produce energy. When L-carnitine is deficient, the concentration of fatty acids in mitochondria is reduced, and thus the production of energy is also reduced. Also, CPT-1, which uses L-carnitine as a substrate, was found to act as an enzyme of reducing the rate of fatty acid oxidation, among various enzymes involved in fatty acid oxidation (Eaton, Prog Lipid Res 41(3): 197-269, 2002).

Caffenine, which is a methylxanthine material known as a positive control group of a lipolysis promoter, shows a lipolytic effect by increasing intracellular cAMP through the inhibition of phosphodiesterase closely associated with lipolysis in adipocytes (Astrup, A. et al., Am J. Clin. Nutr. 51:759, 1990).

Arginine is a natural L-amino acid, which is widely present in nature, and particularly, is contained in the sperm protein of fishes in a relatively large amount. It was reported that the intake of arginine stimulates the secretion of glucagon, which directly accelerates the decomposition of human adipose tissue (Kalkhoff RK, et al., N Engl J Med 289: 465-467). In fact, it was found that, when the blood glucagon concentration is high, the contents of free fatty acid and glycerol are increased.

The extraction of the active ingredients, which are used in the present invention, can be performed using a method suitably selected from among methods known in the art.

The skeletal muscle, which accounts for about 40% of the human body, is a major tissue that depends on the carbohydrate metabolism, and is also a major site that forms insulin resistance in obesity and type 2 diabetes (DeFronzo, 1992). Type 2 diabetes are characterized by abnormal insulin resistance and sugar metabolism in the skeletal muscle, and result in not only the interference of blood glucose maintenance, but also the disturbance of the fat metabolism due to an increase in circulating free fatty acid levels in blood (Reaven et al., 1988), a decrease in fat oxidation in the body (Kelly et al., 1999), and an increase in lipid deposition in various tissues, including the skeletal muscle (Pan et al., 1997).

Skeletal muscles and fatty acids contain glucose carrier Glut4. When the translocation of the glucose carrier (Glut4), which is regulated by insulin, into the cell surface, is stimulated, glucose is imported into cells. Insulin stimulates the translocation of Glut4 toward the cell membrane.

In type 2 diabetes, a defect in the translocation of Glut4 by insulin occurs, and this is also the characteristic of type 2 diabetes. The translocation of Glut4 can also be induced by other stimuli in addition to insulin, and such stimuli include exercise (contraction) (Lundet et al., 1995), hypoxia (Wojtaszewski et al., 1998), and various chemicals (Tsakiridis et al., 1995).

Adiponectin, which is one of typical adipokines secreted from fatty acids, was reported to have anti-obesity action, anti-diabetic action, anti-arteriosclerotic action, and an activity of inhibiting active oxygen production. Adiponectin functions to increase insulin sensitivity so as to lower blood glucose levels, thus preventing diabetes, and in addition, it acts on the liver and muscles to increase AMP kinase activity, so that it shows an anti-obesity effect of promoting fatty acid oxidation and inhibiting fat synthesis.

Adiponectin is a protein, the expression of which is increased with the differentiation of adipocytes, and it is regulated by transcription factors, such as PPAR-γ, C/EBP-α, SREBP1c, liver receptor homolog-1, Krupperl-like factor 7 (KLF7) and the like. In particular, KLF7 is a zinc finger protein reported to be very closely connected with type 2 diabetes. KLF7 is expressed in almost all tissues and is known to induce diabetes by inhibiting the differentiation of adipocytes, inhibiting the secretion of adiponectin and the like and inhibiting the secretion of insulin from pancreatic cells.

The composition according to the present invention is applied in the form of oral drugs and foods to decompose body fat and subcutaneous fat and, at the same time, to restore the expression of Glut4 and adiponectin, which are major bio-markers involved in insulin sensitivity, thus lowering blood glucose levels. This is because the composition of the present invention has the effects of promoting lipolysis and fat combustion and improving and maintaining insulin resistance, through different mechanisms in adipocytes.

In the present invention, the isoflavone is preferably an isoflavone-containing soybean extract, which is contained in an amount of 0.001-30 wt% based on the total weight of the composition.

Also, the composition according to the present invention preferably contains, based on the total weight of the composition, 0.0001-10 wt% of isoflavone and 0.001-40 wt% of L-carnitine.

Moreover, the composition of the present invention contains, based on the total weight of the composition, 0.0001-10 wt% of caffeine and 0.001-40 wt% of arginine.

The contents of the components in the composition of the present invention were determined considering the synergistic effect of the components in the human body and the characteristic of each of the components in terms of safety. Also, the upper limits of the contents of the components were determined considering the molding conditions of a formulation comprising the components.

Accordingly, the present invention provides an oral composition for improving obesity and diabetes, which contains isoflavone, L-carnitine, caffeine and arginine, so that the composition promotes the decomposition of neutral fat in adipocytes, and inhibits the induction of insulin resistance through the promotion of lipolysis and fat burning to restore insulin sensitivity, thus inhibiting blood glucose elevation.

The composition of the present invention can be used as health foods, medical drugs and the like by suitably selecting, in addition to the above-described components, components conventionally used in the art, and then formulating the components in the form of tablets, capsules, soft capsules, pills, granules, drinks, diet bars, chocolates, caramels, confectionaries and the like.

### [Advantageous Effects]

As described above, the composition of the present invention contains isoflavone, carnitine, caffeine and arginine, so that it has the effects of promoting a process of decomposing neutral fat, accumulated in adipocytes, into free fatty acid and glycerol, and in addition, has the effect of promoting a process of burning fatty acid. Moreover, the inventive composition is effective not only in reducing body weight and body fat, but also in treating and improving type 2 diabetes by inhibiting the induction of insulin resistance by caffeine, which has a function of inhibiting the differentiation of adipocytes.

### [Description of Drawings]

FIG. 1 shows the effect of treatment with a mixture of isoflavone, L-carnitine, caffeine and arginine on the change of blood biochemical components in male KK mice.
FIG. 2 shows the effect of treatment with a mixture of isoflavone, L-carnitine, caffeine and arginine on lipolysis in male KK mice.
FIG. 3 shows the effect of treatment with a mixture of isoflavone, L-carnitine, caffeine and arginine on fatty acid oxidation in male KK mice.
FIG. 4 shows the effect of treatment with a mixture of isoflavone, L-carnitine, caffeine and arginine on the inhibition of differentiation of preadipocyte 3T3-L1.
FIG. 5 shows the effect of treatment with a mixture of isoflavone, L-carnitine, caffeine and arginine on the promotion of lipolysis in preadipocyte 3T3-L1.
FIG. 6 shows the effect of treatment with a mixture of isoflavone, L-carnitine, caffeine and arginine on increases in the expressions of adiponectin and Glut4 in preadipocyte 3T3-L1.

### [Best Mode]

Hereinafter, the present invention will be described in further detail with reference to the following test examples. However, it will be obvious to those skilled in the art that these test examples are illustrative only, and the scope of the present invention is not limited thereto.

### Reference Example 1

The epididymal adipose tissue of male KK mice was isolated, and then finely cut with scissors, and 0.1% collagenase (in DMEM without phenol red) was added thereto. Then, the tissue was cultured at 37°C for 2 hours, and then filtered, thus obtaining adipocytes.

### Test Example 1: Effect of treatment with mixture of isoflavone, L-carnitine, caffeine and arginine on body weight reduction in male KK mice

In order to examine the effect of the inventive composition on the lipid metabolism of obesity animals fed with a high fat diet, male KK mice were selected for use in the test. In order to examine the effect of a mixture of isoflavone-containing soybean extract, carnitine, arginine and caffeine (hereinafter, referred to as "ICAC"), 6-week-old mice were acclimated for one week and fed with a high-calorie diet for 3 weeks. Then, the mice were randomly grouped into four test groups, each consisting of 12 animals. The test groups were as follows: (1) a normal-fat diet group; (2) a group fed with normal diet + 20 mg isoflavone + 150 mg L-carnitine; (3) a group fed with normal diet + 600 mg arginine + 25 mg caffeine; and (4) a group fed with normal diet + 20 mg isoflavone + 150 mg L-carnitine + 600 mg arginine + 25 mg caffeine. The test groups were fed with the test diets for 2 weeks. Herein, the test diets were prepared to have a total calorie of 3.1 kcal/g, because the total calorie of the high-calorie diet was 4.7 kcal/g as shown in Table 1 below.

**[Table 1]**

| Ingredients | High calorie diet | Normal diet |
|---|---|---|
| Casein | 18 | 18 |
| Corn starch | 53.4 | 53.4 |
| Corn oil | 20.0 | 2.5 |
| Cellulose powder | 2.5 | 20.0 |
| Mineral mixture | 5 | 5 |
| Vitamin mixture | 1 | 1 |
| Choline bitartrate | 0.1 | 0.1 |

| | | |
|---|---|---|
| 1) Mineral mixture : AIN-93G mineral mixture (g/kg mix). 2) Vitamin mixture: AIN-93G vitamin mixture (g/kg mix). | | |

During the feeding period of the test diets, the diet intake and body weight of the mice were measured three times each week. After completion of the feeding of the test diets, the body weight of the mice was finally measured, and the measurement results of a change in body weight, caused by the test diets, are shown in Table 2 below.

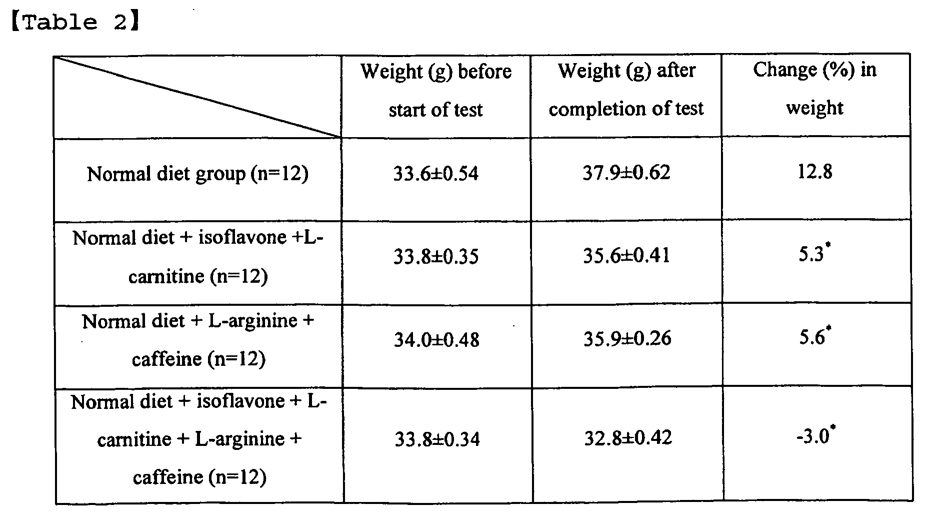

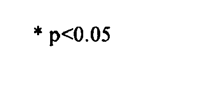

As can be seen in Table 2 above, before the start of the test, there was no difference in body weight between the test groups. However, during the test period, the group fed with isoflavone + L-carnitine and the group fed with L-arginine + caffeine showed increases in body weight of 5.3% and 5.6%, which were significantly lower than 12.8% for the normal diet group. Also, the group fed with isoflavone + L-carnitine + L-arginine + caffeine showed an increase in body weight of -3.0%, which was significantly lower than that of the normal diet group, suggesting that there was a synergistic effect between the components of the mixture. In addition, there was no significant difference in diet intake between the test groups.

### Test Example 2: Evaluation of effect of treatment with mixture of isoflavone, L-carnitine, caffeine and arginine on change of blood biochemical components in male KK mice

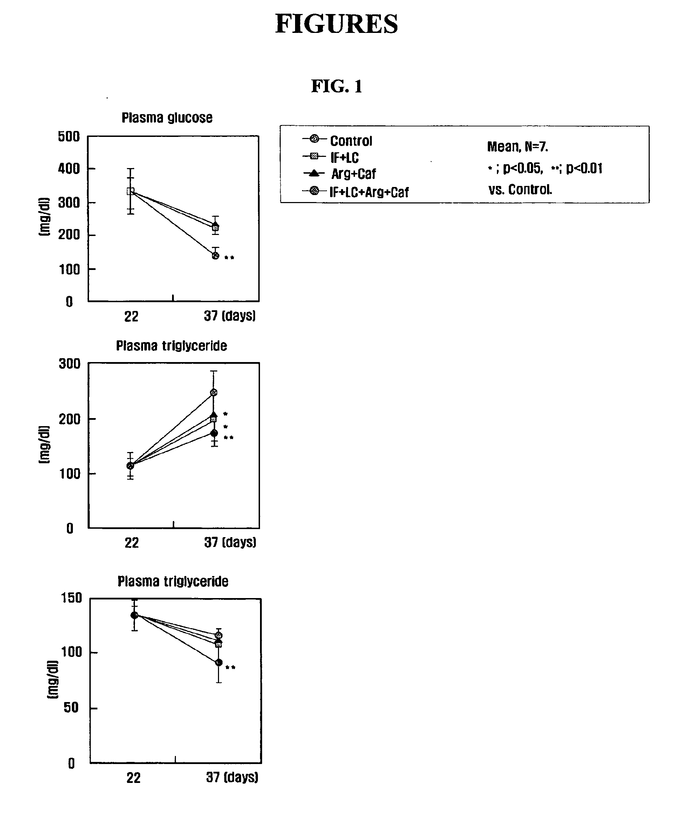

In order to examine the effect of the inventive composition on the lipid metabolism of obesity animals fed with a high fat diet, male KK mice was selected as test models and grouped into four groups: a normal diet group; a group fed with normal diet + isoflavone + L-carnitine; normal diet + L-arginine + caffeine; normal diet + isoflavone + L-carnitine + L-arginine + caffeine. The test diets were fed to the test animals at the same concentrations as in Example 1 for 2 weeks. After completion of diet feeding, the animals were sacrificed, and 2 ml of blood was sampled from the mice using an orbital blood sampling method. The blood sample was centrifuged at 10000 rpm for 10 minutes, and the supernatant (plasma) was isolated and analyzed for plasma glucose, triglyceride and total cholesterol levels using an automatic blood analyzer (H1 system, Technicon, USA). In the glucose and total cholesterol levels, the group fed with isoflavone + L-carnitine and the group fed with L-arginine + caffeine showed no significant decrease compared to the normal diet group, but the group fed with isoflavone + L-carnitine + L-arginine + caffeine showed a significant decrease compared to the normal diet group. In the triglyceride level, the group fed with isoflavone + L-carnitine and the group fed with L-arginine + caffeine showed a significant decrease compared to the normal diet group, and the group fed with isoflavone + L-carnitine + L-arginine + caffeine showed a decrease of 34% compared to the normal diet group. These results are shown in Table 1 below. In FIG. 1, IF indicates isoflavone; LC, L-carnitine; Arg, arginine; and Caf, caffeine.

### Test Example 3: Evaluation of effect of treatment with mixture of isoflavone, L-carnitine, caffeine and arginine on fat tissue in male KK mice

In order to examine the effect of the inventive composition on the lipid metabolism of obesity animals fed with a high fat diet, male KK mice was selected as test models and grouped into four groups: a normal diet group; a group fed with normal diet + isoflavone + L-carnitine; normal diet + L-arginine + caffeine; normal diet + isoflavone + L-carnitine + L-arginine + caffeine. The test diets were fed to the test animals at the same
concentrations as in Example 1 for 2 weeks. After completion of diet feeding, the animals were sacrificed, and the liver, subcutaneous fat, epididymal adipose, peritoneal and retroperitoneal adipose and mesenteric adipose were resected from the animals. The resected tissues were washed with physiological saline and placed on filter paper so as to remove water. Then, the weights of the tissues measured, and the measurement results are shown in Table 3 below.

**[Table 3]**

| | Normal diet group | Normal diet + isoflavone +L- carnitine (n=12) | Normal diet + L- argginine + caffeine (n=12) | Normal diet + isoflavone + L- carnitine + L- arginine + caffeine (n=12) |
|---|---|---|---|---|
| Liver weight (g.100g b.w.) | 3.30±0.34 | 2.94±0.12* | 2.89±0.24* | 2.72±0.32* |
| Liver triglyceride (mg/g liver) | 34.0±4.2 | 27.7±5.3 | 26.2±3.9 | 20.7±7.6* |
| Subcutaneous adipose (g/100g b.w.) | 0.92±0.17 | 0.84±0.06 | 0.78+-0.05 | 0.67±0.12* |
| Epididymal adipose (g/100g b.w.) | 3.45±0.26 | 2.81±0.28* | 2.94±0.18* | 2.21±0.23 * |
| Peritoneal & Retroperitoneal adipose (g/100g b.w.) | 1.43±0.21 | 1.27±0.32 | 1.15±0.21 | 0.82+0.38 * |
| Mesenteric (g/100g b.w.) | 1.77±0.28 | 1.62±0.27 | 1.56±0.31 | 1.31±0.36* |

| | | | | |
|---|---|---|---|---|
| * p<0.05 | | | | |

During the test period, the group fed with isoflavone + L-carnitine and the group fed with L-arginine + caffeine did not show a great decrease in the weight of adipose tissues compared to the normal diet group, but the group fed with isoflavone + L-carnitine + L-arginine + caffeine showed a statistically significant decrease in the weight of adipose tissues compared to the normal diet group. Thus, it was observed that isoflavone, L-carnitine, L-arginine and caffeine showed a synergistic effect on a reduction in body fat in the mice fed with high-calorie diet.

### Test Example 4: Evaluation of effect of treatment with mixture of isoflavone, L-carnitine, caffeine and arginine on lipolysis in male KK mice

20-week-old male KK mice were fed with normal diet, normal diet + 2 mg isoflavone + 15 mg L-carnitine, normal diet + 60 mg L-arginine + 2.5 mg caffeine, and normal diet + 2 mg isoflavone + 15 mg L-carnitine + 60 mg L-arginine + 2.5 mg caffeine, and after 60 minutes, the animals were fed with 50µg/100g b.w. of epinephrine in order to induce lipolysis. At 120 minutes after the feeding of the diets, plasma was collected from the animals of the four test groups according to the method of Test Example 2, and tests for evaluating the effects of the diets on the promotion of decomposition of neutral fat in the adipocytes of the KK mice were performed using the collected plasma. The lipolysis effects were determined by measuring the concentration of glycerol released from the adipocytes into the plasma. The quantification of glycerol was performed using a GPO-trinder kit (Sigma, St. Louis, MO, U.S.A), and the absorbance was measured at 540 nm using an ELISA reader.

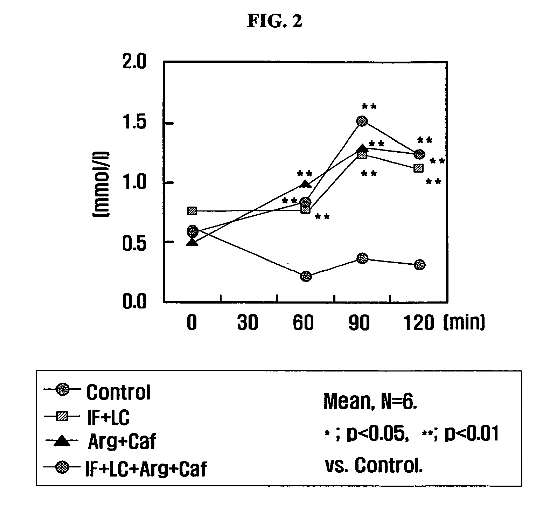

In the results of glycerol quantification, as shown in FIG. 2, the group treated with normal diet + isoflavone + L-carnitine and the group treated with normal diet + L-arginine + caffeine showed increases in fatty acid degradation of about 3.2 times and 3.7 times, respectively, compared to the control group, and the group treated with normal diet + isoflavone + L-carnitine + L-arginine + caffeine showed an increase in fatty acid degradation of about 5.6 times. Also, it could be observed that the amount of glycerol, which was released into blood due to lipolysis, was increased with the passage of time, and the composition of the four components was most effective. In FIG. 2, IF indicates isoflavone; LC, L-carnitine; Arg, arginine; and Caf caffeine.

### Test Example 5: Effect of treatment with mixture of isoflavone, L-carnitine, caffeine and arginine on fatty acid oxidation in male KK mice

In order to evaluate the promotion of decomposition of neutral fat in the adipocytes of KK mice according to the same method as in Test Example 4, clamps were placed around the artery and vein of the muscle tissue of the mice, and at 0 min, 60 min and 90 min from 120 minutes after the diet feeding, 200 ml of blood was sampled from each of the test groups using a syringe (Sarstedt, Leicester, United Kingdom). Plasma was isolated from each of the blood sample. The effects of the test diets on fatty acid oxidation were determined by calculating non-esterified fatty acid (NEFA) uptake. The NEFA amount was quantified using a Wako NEFA C kit (Wako Chemicals Inc., Richmond, VA), the absorbance was measured at 550 nm using an ELISA reader, and the NEFA uptake was calculated from the difference between the imported NEFA amount and the released NEFA amount.

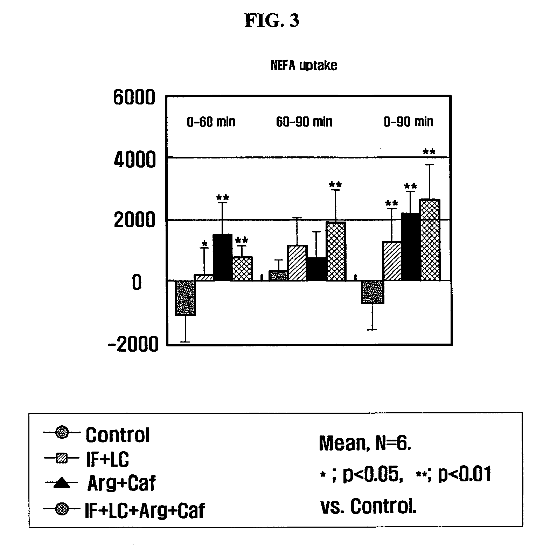

In the results of quantification of NEFA uptake (non-esterified fatty acid uptake), as shown in FIG. 3, the group treated with normal diet + isoflavone + L-carnitine and the group treated with normal diet + L-arginine + caffeine showed increases in NEFA (reuse of degraded fatty acid in fatty acid metabolism in mitochondria) of about 2.7 times and 4.2 times, respectively, compared to the control group, and the group treated with normal diet + isoflavone + L-carnitine + L-arginine + caffeine showed an increase of about 5.2 times. In FIG. 3, IF indicates isoflavone; LC, L-carnitine; Arg, arginine; and Caf, caffeine.

### Test Example 6: Effect of treatment with mixture of isoflavone, L-carnitine, caffeine and arginine on inhibition of 3T3-L1 adipocyte differentiation

### Step 1: Adipocyte cell line and cell differentiation

Mouse undifferentiated 3T3-L1 adipocytes) (purchased from ATCC) were cultured in 10% calf serum-containing DMEM (Dulbecco's modified Eagle's medium, Gibco 1210-0038) in a 10% CO₂ incubator to a confluency of 70% while replacing the medium with a fresh medium at 2-day intervals. For differentiation into adipocytes, the cells were cultured in a medium (containing 10% fetal bovine serum, 0.5 mM 3-isobutyl-1-methyxanthine (Sigma), 1 µM dexamethasone (Sigma) and 167 nM insulin (Novo-Nordisk)) for 48 hours, and then the medium was replaced with a DMEM medium (containing 10% fetal bovine serum and 167 nM insulin), in which the cells were further cultured for 48 hours. Finally, the cells were further cultured in a medium (containing only 10% fetal bovine serum) for 48 hours, thus obtaining differentiated adipocytes.

### Step 2: Effect of treatment with isoflavone, L-carnitine, caffeine and arginine on inhibition of 3T3-L1 adipocyte differentiation

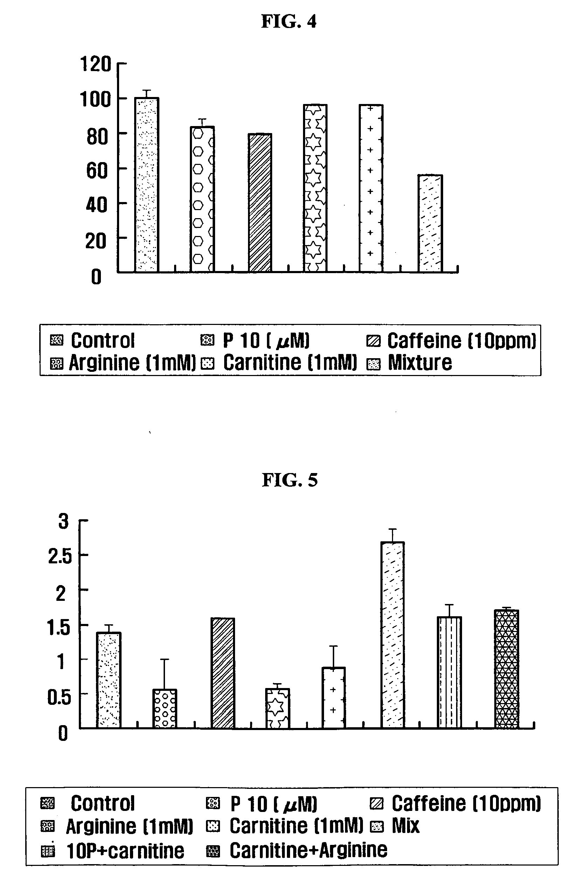

The adipocytes, differentiated in the step 1, were cultured in a medium, containing 5% fatty acid-free calf serum, for 16 hours. On the next day, the cells were washed three times with PBS, and then treated with each of 1 µM isoflavone, 1 mM L-carnitine, 1 mM L-arginine, 10 ppm caffeine and a mixture of 1 µM isoflavone+ 1 mM + 1 mM L-arginine + 10 ppm caffeine. The cells were treated with each of the test materials together with the replacement of medium at 48-hr intervals, and after 8 days, the amount of neutral fat in the cells was measured through Sudan II staining. The measurement results are shown in FIG. 4, in which P10 indicates isoflavone.

As can be seen in FIG. 4, when the adipocytes were treated with the mixture of low concentrations of the four low-concentration components, which are not effective alone or are slightly effective alone, the differentiation of the adipocytes was inhibited by about 40%, suggesting that the four components effectively inhibited the differentiation of the adipocytes.

### Test Example 7: Effect of treatment with mixture of isoflavone, L-carnitine, caffeine and arginine on promotion of lipolysis in 3T3-L1 adipocytes

### Step 1: Adipocyte cell line and cell differentiation

This step was conducted in the same manner as in the step 1 of Test Example 6.

### Step 2: Step 2: Effect of treatment with isoflavone, L-carnitine, caffeine and arginine on promotion of lipolysis in 3T3-L1 adipocytes

The adipocytes, differentiated in the step 1, were cultured in a medium, containing 5% fatty acid-free calf serum, for 8 days. Then, the differentiated adipocytes were washed three times with PBS, and then treated with each of 10 µM isoflavone, 0.5 mM L-carnitine, 1 mM L-arginine, 10 ppm caffeine and a mixture of 10 µM isoflavone 0.5 mM L-carnitine + 1 mM arginine + 10 ppm caffeine for 6 hours. Then, the medium was collected, and the concentration of glycerol in the medium was measured using a GPO-Trinder kit (Sigma diagnostics, St. Louis, MO). The measurement results are shown in FIG. 5, in which P10 indicates isoflavone.

As can be seen in FIG. 5, when the adipocytes were treated with the mixture of low concentrations of the four components, which are not effective alone or are slightly effective alone, the decomposition of neutral fat was about two times higher than that in the control cells. This suggests that, when the four components are used in a mixture, they show a synergistic effect on the decomposition of neutral fat.

### Test Example 8: Effect of treatment with mixture of isoflavone, L-carnitine, caffeine and arginine on increase in expression of adiponectin in 3T3-L1 adipocytes

### Step 1: Adipocyte cell line and cell differentiation

This step was performed in the same manner as in the step 1 of Test Example 6

### Step 2: Effect of treatment with mixture of isoflavone, L-carnitine, caffeine and arginine on increase in expression of adiponectin in 3T3-L1 adipocytes

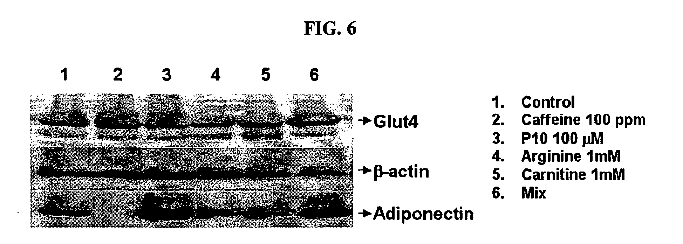

The adipocytes, differentiated in the step 1, were cultured in a medium, containing 5% fatty acid-free calf serum, for 16 hours. On the next day, the cultured cells were washed three times with PBS, and then treated with each of 100 µM isoflavone, 1 mM L-carnitine, 1 mM L-arginine, 100 ppm caffeine and a mixture of 100 µM isoflavone + 1 mM L-carnitine + 1 mM L-arginine + 100 ppm caffeine. After 24 hours of cell incubation, a protein was isolated from each of the media and subjected to Western blot analysis in order to examine a change in the expression of adiponectin. The analysis results are shown in FIG. 6. As can be seen in FIG. 6, the expression of adiponectin was increased during the differentiation of the adipocytes. Thus, the inhibition of differentiation of the adipocytes led to a decrease in the expression of adiponectin. Because caffeine inhibits the differentiation of adipocyte differentiation, the cells treated with caffeine showed a decrease in the expression of adiponectin. However, when the adipocytes were treated with the mixture of isoflavone + L-carnitine + L-arginine + caffeine, the differentiation of the adipocytes was inhibited, but the expression of adiponectin was maintained. In FIG. 6, P10 indicates isoflavone.

This effect is believed to be because the four components were used in a mixture. That is, it was found that, when adipocytes were treated with the mixture of the four components, and the accumulation of fat in the adipocytes was inhibited, the consumption of energy in other tissues was continuously promoted. Thus, the use of the four components in a mixture is effective in improving type 2 diabetes caused by insulin resistance, which can occur when a substance having the same mechanism as that of caffeine is used alone.

### Test Example 9: Effect of treatment with mixture of isoflavone, L-carnitine, caffeine and arginine on increase in expression of Gult4 in 3T3-L1 adipocytes

### Step 1: Adipocyte cell line and cell differentiation

This step was performed in the same manner as in the step 1 of Test Example 6

### Step 2: Effect of treatment with mixture of isoflavone, L-carnitine, caffeine and arginine on increase in expression of Glut4 in 3T3-L1 adipocytes

The adipocytes, differentiated in the step 1, were cultured in a medium, containing 5% fatty acid-free calf serum, for 16 hours. On the next day, the cultured cells were washed three times with PBS, and then treated with each of 100 µM isoflavone, 1 mM L-carnitine, 1 mM L-arginine, 100 ppm caffeine and a mixture of 100 µM isoflavone + 1 mM L-carnitine + 1 mM L-arginine + 100 ppm caffeine. After 24 hours of cell incubation, a protein was isolated from each of the media and subjected to Western blot analysis in order to examine a change in the expression of Glut4. The analysis results are shown in FIG. 6.

As can be seen in FIG. 6, when the adipocytes were treated with the mixture of low concentrations of isoflavone + L-carnitine + L-arginine + caffeine, which are not effective alone or are slightly effective alone, the expression of Glut4 in the treated adipocytes was significantly increased compared to that in the control cells. Also, it was found that, when the amount of house keeping protein β-actin was considered, the mixture of isoflavone + L-carnitine + L-arginine + caffeine would be effective in overcoming insulin resistance, which can occur when β-actin is used to treat obesity.

### [Industrial Applicability]

As described above, the inventive composition for the improvement of obesity and diabetes is effective not only in reducing body weight and body fat, but also in treating and improving type 2 diabetes. Accordingly, it is very useful in the food and drug industries.

## Claims

1. Composition containing isoflavone, L-carnitine, caffeine and arginine as active ingredients, for use in the treatment of obesity and diabetes.

2. The composition for use according to Claim 1, wherein said isoflavone is isoflavone-containing soybean extract.

3. The composition for use according to Claim 2, wherein the soybean extract is contained in an amount of 0.001-30 wt% based on the total weight of the composition.

4. The composition for use according to Claim 1, wherein said isoflavone and L-carnitine are contained in amounts of 0.0001-10 wt% and 0.001-40 wt%, respectively, based on the total weight of the composition.

5. The composition for use according to Claim 1, wherein said caffeine and arginine are contained in amounts of 0.0001-10 wt% and 0.001-40 wt%, respectively, based on the total weight of the composition.

6. The composition for use according to Claim 1, wherein the active ingredients serve to inhibit the induction of insulin resistance by the promotion of lipolysis and fat burning, so as to restore insulin sensitivity, thus inhibiting blood glucose elevation.

7. The composition for use according to Claim 6, wherein the composition serves to increase the expressions of Glut4 and adiponectin genes.

8. The composition for use according to Claim 1, wherein the composition is used in health food.

9. The composition for use according to Claim 1, wherein the composition is used in drugs.

10. The composition for use according to Claim 1, wherein said composition is formulated in the form of tablets, capsules, soft capsules, pills, granules, drinks, diet bars, chocolates, caramels or confectionaries.

## Patentansprüche

1. Zusammensetzung enthaltend Isoflavon, L-Carnitin, Coffein und Arginin als Wirkstoffe zur Verwendung bei der Behandlung von Fettleibigkeit und Diabetes.

2. Zusammensetzung zur Verwendung nach Anspruch 1, wobei das Isoflavon Isoflavonhaltiger Sojabohnenextrakt ist.

3. Zusammensetzung zur Verwendung nach Anspruch 2, wobei der Sojabohnenextrakt in einer Menge von 0,001 - 30 Gew.-%, bezogen auf das Gesamtgewicht der Zusammensetzung, enthalten ist.

4. Zusammensetzung zur Verwendung nach Anspruch 1, wobei das Isoflavon und L-Carnitin in Mengen von 0,0001 - 10 Gew.-% bzw. 0,001 - 40 Gew.-%, bezogen auf das Gesamtgewicht der Zusammensetzung, enthalten sind.

5. Zusammensetzung zur Verwendung nach Anspruch 1, wobei das Coffein und Arginin in Mengen von 0,0001 - 10 Gew.-% bzw. 0,001 - 40 Gew.-%, bezogen auf das Gesamtgewicht der Zusammensetzung, enthalten sind.

6. Zusammensetzung zur Verwendung nach Anspruch 1, wobei die Wirkstoffe dazu dienen, die Induktion einer Insulinresistenz durch die Förderung von Lipolyse und Fettverbrennung zu hemmen, so dass die Insulinempfindlichkeit wieder hergestellt wird, und somit ein Anstieg der Blutglucose gehemmt wird.

7. Zusammensetzung zur Verwendung nach Anspruch 6, wobei die Zusammensetzung dazu dient, die Expression von Glut4 und Adiponectin-Genen zu erhöhen.

8. Zusammensetzung zur Verwendung nach Anspruch 1, wobei die Zusammensetzung in Gesundheitsnahrung verwendet wird.

9. Zusammensetzung zur Verwendung nach Anspruch 1, wobei die Zusammensetzung in Arzneimitteln verwendet wird.

10. Zusammensetzung zur Verwendung nach Anspruch 1, wobei die Zusammensetzung in Form von Tabletten, Kapseln, Weichkapseln, Pillen, Körnchen, Getränken, Diätriegeln, Pralinen, Karamellen oder Konfekt formuliert ist.

## Revendications

1. Composition contenant de l'isoflavone, de la L-carnitine, de la caféine et de l'arginine en tant qu'ingrédients actifs, pour utilisation dans le traitement de l'obésité et du diabète.

2. Composition pour utilisation selon la revendication 1, dans laquelle ladite isoflavone est un extrait de soja contenant de l'isoflavone.

3. Composition pour utilisation selon la revendication 2, dans laquelle l'extrait de soja est contenu en une quantité de 0,001-30% en poids par rapport au poids total de la composition.

4. Composition pour utilisation selon la revendication 1, dans laquelle l'isoflavone et la L-carnitine sont contenues en des quantités de 0,0001-10 % en poids et 0,001-40 % en poids, respectivement, par rapport au poids total de la composition.

5. Composition pour utilisation selon la revendication 1, dans laquelle ladite caféine et ladite arginine sont contenues en des quantités de 0,0001-10 % en poids et 0,001-40 % en poids, respectivement, par rapport au poids total de la composition.

6. Composition pour utilisation selon la revendication 1, dans laquelle les ingrédients actifs servent à inhiber l'induction de la résistance à l'insuline par la promotion de la lipolyse et de la brûlure des graisses, de façon à restaurer une sensibilité à l'insuline, inhibant ainsi une élévation du glucose sanguin.

7. Composition pour utilisation selon la revendication 6, dans laquelle la composition sert à augmenter l'expression des gènes *Glut4* et *adiponectin.*

8. Composition pour utilisation selon la revendication 1, dans laquelle la composition est utilisée dans des aliments naturels.

9. Composition pour utilisation selon la revendication 1, dans laquelle la composition est utilisée dans des médicaments.

10. Composition pour utilisation selon la revendication 1, dans laquelle ladite composition est formulée sous formes de comprimés, capsules, capsules molles, pilules, granules, boissons, barres diététiques, chocolats, caramels ou confiseries.
